# EUROPEAN PATENT SPECIFICATION

(11) **EP 2 680 879 B1**
(45) Date of publication and mention of the grant of the patent: **06.01.2021**
(21) Application number: 12754246.2
(22) Date of filing: 02.03.2012
(51) Int. Cl.: A61K 38/47, C12N 9/24, A61P 43/00, A61K 38/46, A61K 9/00, A61K 47/65, C07K 14/65, C07K 7/08, A61K 38/30

(54) **PEPTIDE LINKERS FOR POLYPEPTIDE COMPOSITIONS AND METHODS FOR USING SAME**
PEPTIDLINKER FÜR POLYPEPTIDZUSAMMENSETZUNGEN UND ANWENDUNGSVERFAHREN DAFÜR
LIEURS PEPTIDIQUES POUR COMPOSITIONS DE POLYPEPTIDE ET PROCÉDÉS POUR LES UTILISER

(30) Priority: 04.03.2011 US 201161449225 P; 25.06.2011 WO PCT/US2011/041928; 25.06.2011 US 201113168969
(43) Date of publication of application: 08.01.2014
(73) Proprietor: Shire Human Genetic Therapies, Inc., Lexington MA 02421 (US)
(72) Inventor: MARTINI, Paolo, Boston, MA 02118 (US); CONCINO, Michael, Bolton, MA 01740 (US)
(74) Representative: Carpmaels & Ransford LLP
(86) International application number: PCT/US2012/027561
(87) International publication number: WO 2012/122042

(56) References cited:
- WO-A2-01/05824
- WO-A2-2007/141346
- WO-A2-2009/137721
- WO-A2-2011/163652
- US-A1- 2007 061 916
- US-A1- 2008 279 765
- US-A1- 2010 075 906
- US-A1- 2010 233 095
- US-A9- 2008 171 852
- GEORGE R A ET AL: "An analysis of protein domain linkers: their classification and role in protein folding", PROTEIN ENGINEERING, OXFORD UNIVERSITY PRESS, SURREY, GB, vol. 15, no. 11, 1 November 2002 (2002-11-01), pages 871-879, XP002374925, ISSN: 0269-2139, DOI: 10.1093/PROTEIN/15.11.871
- Carolyn S. Sinow: "Construction of an IGF-NAGLU Fusion Protein for Treatment of Sanfilippo B Syndrome", , 2008, XP002735889, Retrieved from the Internet: URL:https://www.usc.edu/CSSF/History/2008/ Projects/S0417.pdf [retrieved on 2015-02-12]
- LEBOWITZ J H ET AL: "Glycosylation-independent targeting enhances enzyme delivery to lysosomes and decreases storage in mucopolysaccharidosis type VII mice", PROCEEDINGS OF THE NATIONAL ACADEMY OF SCIENCES, NATIONAL ACADEMY OF SCIENCES, US, vol. 101, no. 9, 2 March 2004 (2004-03-02) , pages 3083-3088, XP002285806, ISSN: 0027-8424, DOI: 10.1073/PNAS.0308728100
- VISHNU PRIYANKA REDDY CHICHILI ET AL: "Linkers in the structural biology of protein-protein interactions", PROTEIN SCIENCE, vol. 22, no. 2, 8 February 2013 (2013-02-08), pages 153-167, XP055169244, ISSN: 0961-8368, DOI: 10.1002/pro.2206
- ROBINSON C R ET AL: "Optimizing the stability of single-chain proteins by linker length and composition mutagenesis", PROCEEDINGS OF THE NATIONAL ACADEMY OF SCIENCES, NATIONAL ACADEMY OF SCIENCES, US, vol. 95, no. 11, 26 May 1998 (1998-05-26), pages 5929-5934, XP002333428, ISSN: 0027-8424, DOI: 10.1073/PNAS.95.11.5929
- DATABASE UNIPROT [Online] 31 October 2006 ARAKI ET AL., XP003032875 Database accession no. Q20IN6_PSECI
- EGERER ET AL.: 'Secreted antiviral entry inhibitory (SAVE) peptides for gene therapy of HIV infection' MOL. THER. vol. 19, no. 7, 01 March 2011, pages 1236 - 1244, XP055137012
- WRIGGERS ET AL.: 'Control of protein functional dynamics by peptide linkers' BIOPOLYMERS vol. 80, no. 6, 2005, pages 736 - 746, XP008117818

## Description

### FIELD OF THE INVENTION

The present inventions are directed to novel peptide linkers and polypeptide compositions comprising such linkers (e.g., chimeric polypeptides), compositions for their use in therapy, and methods of preparing the same.

### BACKGROUND OF THE INVENTION

Lysosomal storage disorders represent a group of more than forty rare and inherited metabolic disorders caused by the deficiency or inactivity of specific lysosomal enzymes. In particular, lysosomal storage disorders are caused by the deficiency or inactivity of the lysosomal enzymes which catalyze the stepwise metabolism of lipids or complex glycoproteins known as glycosaminoglycans. As a result of this metabolic deficiency, metabolic precursors progressively accumulate in the cells, tissues and, in particular, the cellular lysosomes of affected subjects. This protein accumulation causes a permanent, progressive cellular damage which affects the appearance, physical abilities, organ and system functioning and, in most cases, the mental development of affected subjects. Although the enzyme deficiencies affect every tissue, a patient's clinical expression may frequently vary depending, for example, on the degree of enzyme deficiency or impairment. The lysosomal storage disorder may also be associated with some degree of neuronal cell loss, predominantly resulting in neurological symptoms, including, mental retardation, severe motor impairments, physical disability, a decreased lifespan and/or combinations of the foregoing.

There are no cures for the lysosomal storage disorders, and treatment is often palliative, offered to subjects primarily to improve their quality of life. Enzyme replacement therapy (ERT) has been a useful therapeutic option for subjects with lysosome storage disorders. ERT generally involves the parenteral administration of natural or recombinantly-derived proteins and/or enzymes to a patient. Approved therapies are administered to patients intravenously and are generally effective in treating the somatic or peripheral symptoms of the underlying enzyme deficiency. To effectively treat lysosomal storage disorders, the administered therapeutic agent (e.g., the deficient lysosomal enzyme) must distribute into the affected cells and tissues after being infused into a patient's bloodstream.

To achieve distribution of the requisite enzymes into affected cells and tissues, the enzymes are generally targeted to specific cell-surface receptors that transport the enzymes into the cells through receptor-mediated endocytosis. For example, in Gaucher's disease, the deficient enzyme, glucocerebrosidase, is targeted to the appropriate cells through the binding of exposed mannose residues on the enzyme to the mannose receptor, which is abundantly expressed on target cells (reticuloendotheilial cells). In cells that lack the mannose receptor, use of the insulin-like growth factor/cation-independent mannose- 6-phosphate receptor (IGF-II/CI-MPR) has been proposed for delivery of deficient lysozymes to cells (Kornfeld, S., 1987 Biochem Soc Trans 18:367-374). The IGF-II/CI-MPR receptor is present on the surface of many mammalian cell types and thus provides a means by which to target proteins containing the receptor ligand (e.g., IGFII or mannose-6 phosphate) to a wide variety of cells and tissues, including the central nervous system. However, despite some knowledge of how to target missing lysosomal enzymes to appropriate tissues, there are still no effective therapies for many lysosomal storage disorders (e.g., Sanfilippo syndrome, Farber's disease, and the like). Thus, there remains a need in the art for compositions, particularly compositions that can be administered parenterally, and methods useful for directing agents to the necessary tissues to treat diseases (e.g., lysosomal storage diseases).
WO2009/137721 discloses compositions and methods for efficient lysosomal targeting based on Glycosylation Independent Lysosomal Targeting (GILT) technology. Egerer et al. (2011, Mol. Ther., vol. 19, no. 7, pages 1236 - 1244) discloses secreted antiviral entry inhibitory peptides for gene therapy of HIV infection.

### SUMMARY OF THE INVENTION

There is a need for compositions and methods that facilitate the transport and delivery of functional therapeutic agents (e.g., proteins, polypeptides) to the desired tissues. Such compositions and methods may be useful in the treatment of a number of diseases or disorders and, in particular, in the treatment of lysosomal storage disorders, like Sanfilippo disease. Described herein are novel compositions comprising peptide linkers, polypeptide compositions comprising polypeptides joined by the peptide linkers and related polynucleotides, vectors, cells and pharmaceutical compositions. Described linker sequences operably join two peptides/polypeptides of interest such that the expression and activity (e.g., receptor binding and/or enzyme activity) of the polypeptides connected by the linkers are durable and optimal. The polypeptide compositions comprising the peptide linkers facilitate the targeted delivery of polypeptides/proteins of interest to particular cells and/or tissues.

Accordingly, an embodiment of the invention provides for a polypeptide composition comprising a first peptide, a second peptide and a linker consisting of one to four sequential repeats of the amino acid sequence of SEQ ID NO. 1 (GAPGGGGGAAAAAGGGGG), and optionally the amino acid sequence glycine alanine proline (GAP) at the C terminus, wherein the linker is disposed between the first peptide and the second peptide. In some embodiments, the linker of the polypeptide composition comprises three sequential or tandem repeats of SEQ ID NO. 1 and, in some embodiments, the linkers further comprise the amino acid sequence glycine alanine proline (GAP) at the C-terminus of SEQ ID NO. 1. In certain embodiments, the first peptide of the polypeptide composition comprises the amino acid sequence of SEQ ID NO. 4. In still other embodiments the second peptide comprises a receptor binding domain and, in further embodiments, the second peptide comprises the amino acid sequence of SEQ ID NO. 6.

In certain embodiments, a polypeptide composition comprises a first peptide comprising the amino acid sequence of SEQ ID NO. 4; a second peptide comprising the amino acid sequence of SEQ ID NO. 6; and a linker consisting of one to four sequential repeats of the amino acid sequence of SEQ ID NO. 1, and optionally the amino acid sequence glycine alanine proline (GAP) at the C terminus, wherein the linker is disposed between the first peptide and the second peptide. In some embodiments, the linker comprises three sequential repeats of SEQ ID NO. 1. In other embodiments, the linkers can further comprise the amino acid sequence glycine alanine proline (GAP) at the C-terminus of the SEQ ID NO. 1.

In some embodiments, a polypeptide composition comprises a first peptide comprising the amino acid sequence of SEQ ID NO. 4; a second peptide comprising the amino acid sequence of SEQ ID NO. 6; and a linker consisting of the amino acid sequence of SEQ ID NO. 2 disposed between the first peptide and second peptide.

The invention also provides for polypeptide linker composition of one to four sequential repeats of the amino acid sequence of SEQ ID NO: 1 (GAPGGGGGAAAAAGGGGG) and optionally the amino acid sequence glycine alanine proline (GAP) at the C terminus.

In some embodiments, the polypeptide linker consists of the amino acid sequence of SEQ ID NO. 1.

In further embodiments, the C-terminus of the above polypeptide linker compositions further consists of three contiguous amino acids comprising the amino acid sequence glycine alanine proline (GAP).

In some embodiments, a polypeptide linker consists of the amino acid sequence of SEQ ID NO. 2 (GAPGGGGGAAAAAGGGGGGAPGGGGGAAAAAGGGGGGAPGGGGGAAAAAGGGGGGAP).

In other embodiments, the invention also provides for polynucleotides that encode the polypeptide linkers and/or polypeptide compositions described herein. Thus, some embodiments provide a polynucleotide encoding a polypeptide linker consisting of the amino acid sequence of one to four sequential repeats of SEQ ID NO. 1 and optionally the amino acid sequence glycine alanine proline (GAP) at the C-terminus, optionally wherein the linker consists of the amino acid sequence of SEQ ID NO. 2.

In other embodiments, a polynucleotide encodes a polypeptide composition comprising the amino acid sequence of a first peptide; a second peptide; and a linker consisting of the one to four sequential repeats of the amino acid sequence of SEQ ID NO. 1, and optionally the amino acid sequence glycine alanine proline (GAP) at the C terminus, wherein the linker is disposed between the first peptide and the second peptide. In certain embodiments, the linker of the polypeptide composition consists of three sequential repeats of SEQ ID NO. 1. In further embodiments of the foregoing polynucleotides, the C-terminus of the linker of the polypeptide compositions further comprises the amino acid sequence glycine alanine proline (GAP), and the polynucleotide encodes the GAP amino acid sequence.

In certain embodiments, a polynucleotide encodes a first peptide of the polypeptide composition that comprises the amino acid sequence of SEQ ID NO. 4. In other embodiments, the polynucleotide encodes a second peptide of the polypeptide composition that comprises a receptor binding domain and, in some embodiments, the second peptide comprises the amino acid sequence of SEQ ID NO. 6.

Also described herein are expression vectors comprising the aforementioned polynucleotides which encode the polypeptide compositions described herein. Other embodiments described herein relate to recombinant cells comprising the foregoing polynucleotides and expression vectors.

In addition, the invention provides for pharmaceutical compositions comprising the polypeptide compositions, as defined in the claims, and a pharmaceutically acceptable carrier. For example, in some embodiments, the pharmaceutical composition comprises polypeptide compositions comprising a first peptide comprising the amino acid sequence of SEQ ID NO. 4 and a second peptide comprising the amino acid sequence of SEQ ID NO. 6. The linker disposed between the first peptide and second peptide consists of one to four sequential repeats of SEQ ID NO. 1, and optionally the amino acid sequence glycine alanine proline (GAP) at the C terminus. In some of these embodiments, the linker consists of the amino acid sequence of SEQ ID NO. 2.

In certain embodiments, the invention also provides for pharmaceutical compositions comprising the polynucleotides, as defined in the claims, and a pharmaceutically acceptable carrier. The invention further provides for pharmaceutical compositions comprising the recombinant cells, as defined in the claims, and a pharmaceutically acceptable carrier.

Also described herein are methods of producing a polypeptide composition, the method comprising culturing the recombinant cells described herein under conditions suitable for the expression of the polypeptide.

Methods of delivering a therapeutic polypeptide to a subject in need thereof are also described herein.

In some embodiments, the invention provides a polypeptide composition for use in a method of delivering a therapeutic polypeptide to a subject in need thereof wherein the polypeptide composition comprises a first peptide comprising the amino acid sequence of SEQ ID NO. 4; a second peptide comprising the amino acid sequence of SEQ ID NO. 6; and a linker consisting of one to four sequential repeats of the amino acid sequence of SEQ ID NO. 1 and optionally the amino acid sequence glycine alanine proline (GAP) at the C terminus, wherein the linker is disposed between the first peptide and the second peptide. In some embodiments, the linker further comprises GAP at the C-terminus of SEQ ID NO. 1. In certain embodiments, the linker comprises the amino acid sequence of SEQ ID NO. 2.

In some embodiments, the invention also provides one or more expression vectors, as defined in the claims, or one or more recombinant cells, as defined in the claims, for use in a method of delivering a therapeutic polypeptide to a subject in need thereof, wherein the first peptide is a therapeutic agent and the second peptide is a targeting agent.

Also described herein are the pharmaceutical compositions, as defined in the claims, for use in methods of treating a lysosomal storage disease, wherein the first peptide is a lysosomal enzyme and the second peptide is a cell surface receptor ligand protein. In certain embodiments, the lysosomal storage disease is Sanfilippo syndrome.

In other embodiments, the invention provides a pharmaceutical composition for use in a method of treating Sanfilippo syndrome comprising a pharmaceutical composition comprising a polypeptide composition comprising a first peptide comprising the amino acid sequence of SEQ ID NO. 4; a second peptide comprising the amino acid sequence of SEQ ID NO. 6; and a linker consisting of one to four sequential repeats of the amino acid sequence of SEQ ID NO. 1, and optionally the amino acid sequence glycine alanine proline (GAP) at the C terminus, wherein the linker is disposed between said first peptide and said second peptide. In certain embodiments, the linker further comprises the amino acid sequence gap at the C-terminus of the linker. In other embodiments of the method, the linker consists of the amino acid sequence of SEQ ID NO. 2.

In certain embodiments, the pharmaceutical compositions are administered parenterally.

The above discussed and many other features and attendant advantages of the present invention will become better understood by reference to the following detailed description of the invention when taken in conjunction with the accompanying examples.

### BRIEF DESCRIPTION OF THE DRAWINGS

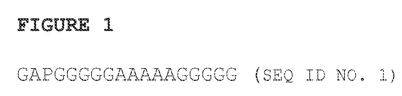

FIG. 1 illustrates the amino acid sequence of a peptide linker comprising a glycine alanine proline (GAP) sequence joined to a glycine alanine glycine (GAG) repeat sequence (SEQ ID NO. 1).

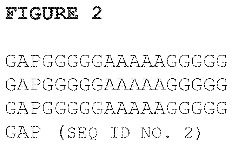

FIG. 2 illustrates the amino acid sequence of a peptide linker comprising three sequential or tandem repeats of SEQ ID NO. 1 joined at the C-terminus to a GAP sequence (SEQ ID NO. 2).
FIG. 3A illustrates the α-N-acetylglucosaminidase-insulin-like growth factor (NaGlu-IGFII) construct joined by a GAP linker. FIG 3B illustrates a NaGlu-IGFII construct joined by the linker of SEQ ID NO. 2 (NaGlu-GAG₃- IGFII).

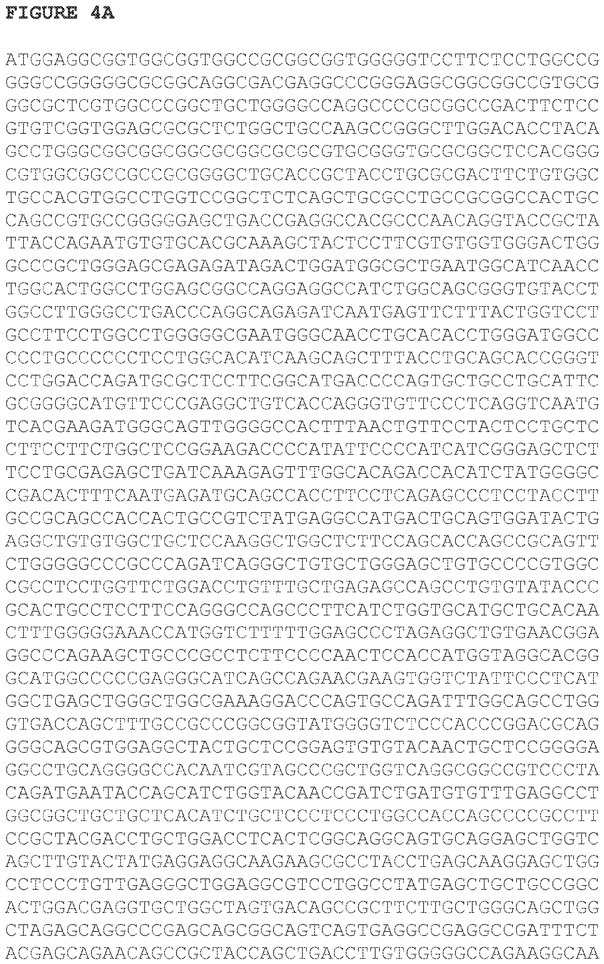

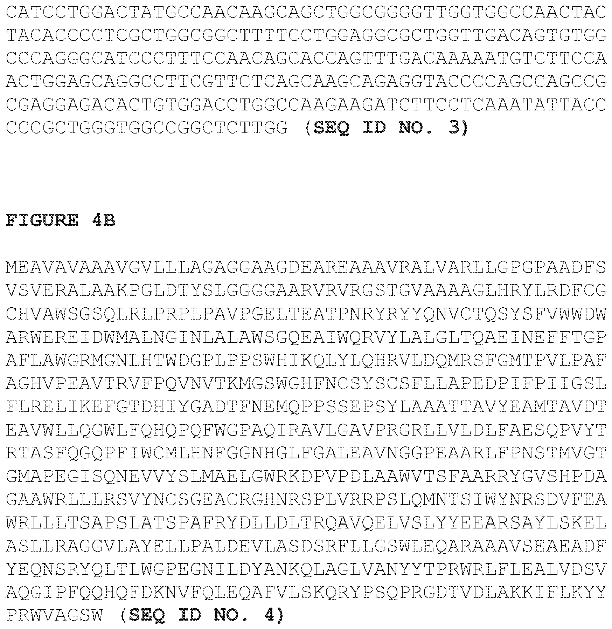

FIG. 4A illustrates the nucleotide sequence of human NaGlu (SEQ ID NO. 3). FIG. 4B illustrates the amino acid sequence of human NaGlu (SEQ ID NO. 4).

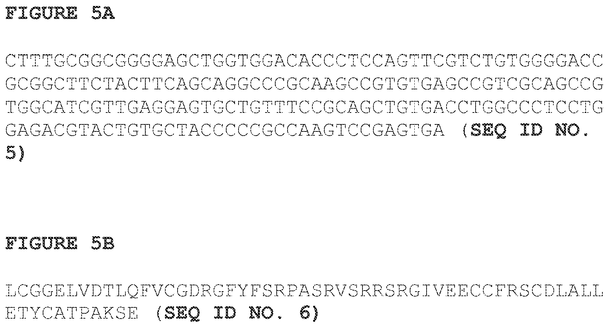

FIG. 5A illustrates the nucleotide sequence of human IGFII (SEQ ID NO. 5) FIG. SB illustrates the amino acid sequence of human IGFII (SEQ ID NO. 6).

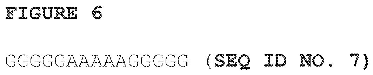

FIG. 6 illustrates the amino acid sequence of a peptide linker comprising a GAG repeat sequence (SEQ ID NO. 7).

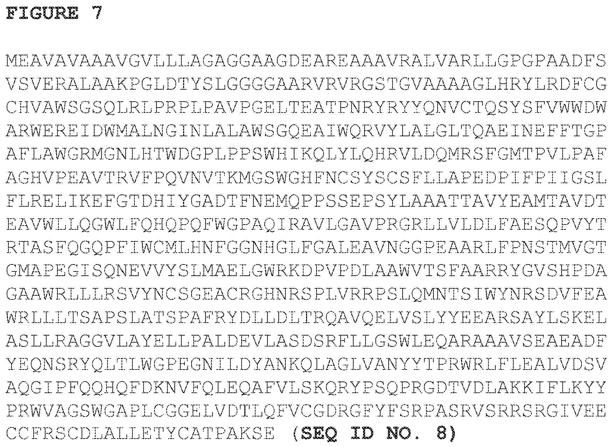

FIG. 7 illustrates the amino acid sequence of the NaGlu-IGFII construct illustrated in FIG 3A (SEQ ID NO. 8) .
FIG. 8 illustrates the amino acid sequence of the NaGlu-IGFII construct illustrated in FIG. 3B (SEQ ID NO. 9) .
FIG. 9 illustrates a comparison of the activity levels of two different NaGlu-IGFII protein constructs (NaGlu-GAG₃-IGFII and NaGlu-IGFII) in HT1080 cells and demonstrates that, compared to wild-type NaGlu, NaGlu-GAG₃- IGFII has very high levels of activity, while NaGlu-IGFII has very little.
FIG. 10 illustrates the expression of NaGlu-GAG₃-IGFII and NaGlu-IGFII polypeptides by western blot and shows that the NaGlu-IGFII protein underwent degradation while wild-type NaGlu and NaGlu-GAG₃-IGFII did not.
FIG. 11 illustrates the uptake of the NaGlu-GAG₃-IGFII polypeptide by human fibroblast cells (HF1156) and demonstrates that NaGlu-GAG₃-IGFII was readily taken-up by human cells.

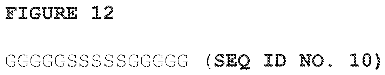

FIG. 12 illustrates the amino acid sequence of a peptide linker (SEQ ID NO. 10).

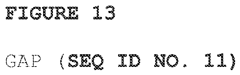

FIG. 13 illustrates the amino acid sequence of a peptide linker (SEQ ID NO. 11).

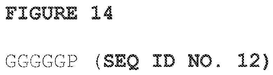

FIG. 14 illustrates the amino acid sequence of a peptide linker (SEQ ID NO. 12).

### DETAILED DESCRIPTION OF THE INVENTION

Compositions are described herein that provide a means to make (e.g., design, engineer) chimeric or fusion polypeptides. The polypeptide compositions can also provide means of facilitating the delivery of agents (e.g., polypeptides/peptides, proteins and/or enzymes) to cells, tissues or organs of interest. In particular, the compositions and methods can be used to selectively deliver agents to an appropriate tissue of a subject in need thereof, thereby treating a disease or disorder. These therapeutic compositions can be polynucleotides or polypeptides that comprise a therapeutic agent (e.g., protein/enzyme) joined to a targeting agent (e.g., cell receptor ligand protein) by a linker sequence that allows for the proper expression, folding and activity of the therapeutic and/or targeting agent. In some aspects, the therapeutic composition comprises a lysosomal protein or enzyme connected by the linker sequence to a cell-surface receptor ligand protein. The therapeutic polypeptide composition can be used to treat disorders such as lysosomal storage diseases.

As used herein, the phrase "lysosomal storage disorder" or lysosomal storage disease" refers to a class of inherited diseases related to the aberrant expression of or deficiency of one or more lysosomal enzymes. These enzyme deficiencies result in detrimental accumulation of metabolic products in the lysosomes of affected subjects. Representative lysosomal storage disorders include aspartylglucosaminuria, cholesteryl ester storage disease, cystinosis, Danon disease, Fabry disease, Farber's disease, fucosidosis, falactosialidosis types I/II, Gaucher disease types 1, 2, 3, globoid cell leucodystrophy/Krabbe disease, glycogen storage disease II/Pompe disease, GMl-gangliosidosis types I/III, GM2-gangliosidosis type I/Tay-Sachs disease, GM2-gangliosidosis type II/Sandhoff disease, α-mannosidosis types I/II, β-mannosidosis, metachromatic leukodystrophy (MLD), mucolipidosis type I/sialidosis types I/II, mucolipidosis types II/III, mucolipidosis type III pseudo-Hurler polydystrophy, mucopolysaccharidosis (e.g. types I, II, IIIA, IIIB, IIIC, IIID, IVA, IVB, VI, VII and IX), multiple sulphatase deficiency, neuronal ceroid lipofuscinosis (e.g. Batten, infantile, late infantile and adult), Niemann-Pick disease (e.g. types A, B, Cl, C2), Schindler disease types I/II, sialic acid storage disease, Sanfilippo disease, and Wolman's disease (acid lipase deficiency). In one aspect of the invention, the compositions comprise a therapeutic agent whose deficiency is linked to a lysosomal storage disorder.

Polypeptide composition and polynucleotides encoding the polypeptide compositions are described herein, in which the polypeptide compositions comprise a first and second peptide/polypeptide, connected by a linker sequence disclosed herein. The inventors have surprisingly found that a linker comprising one or more sequential or tandem repeats of SEQ ID NO. 1 (GAPGGGGGAAAAAGGGGG), connecting two protein sequences (e.g., a first polypeptide and a second polypeptide) results in the production of a polypeptide that is well-expressed and highly active (e.g., biologically active). As used herein, the term "polypeptide" or "peptide" refers a polymer of amino acid residues typically joined exclusively by peptide bonds, that can be produced naturally (e.g., isolated, essentially purified or purified) or synthetically (e.g., by chemical synthesis). A polypeptide produced by expression of a non-host DNA molecule is a "heterologous" peptide or polypeptide. An "amino acid residue" comprising the polypeptide can be a natural or non-natural amino acid residue linked by peptide bonds and/or bonds different from peptide bonds. The amino acid residues can be in D- configuration or L-configuration. In some aspects, the polypeptides referred to herein are proteins, peptides or fragments thereof produced by the expression of recombinant nucleic acid. In some embodiments, the polypeptide compositions described herein comprise two polypeptides connected by a linker sequence (e.g., SEQ ID NO. 2), in which one of the two polypeptides is a peptide that can be administered to treat a disease or a disorder (e.g., a therapeutic peptide) and the other polypeptide is peptide that can be used to deliver a therapeutic peptide to a target cell, tissue or organ (e.g., a targeting peptide).

The linker or polypeptide linker described herein refers to a peptide sequence designed to connect (e.g., join, link) two protein sequences, wherein the linker peptide sequence is typically not disposed between the two protein sequences in nature. In the context of the present invention, the phrase "linked" or "joined" or "connected" generally refers to a functional linkage between two contiguous or adjacent amino acid sequences to produce a polypeptide that generally does not exist in nature. In certain embodiments, linkage may be used to refer to a covalent linkage of, for example, the amino acid sequences of the one or more therapeutic peptide agents and the one or more targeting agents (e.g., binding or receptor ligand peptides). Generally, linked proteins are contiguous or adjacent to one another and retain their respective operability and function when joined. Peptides comprising the chimeric polypeptides disclosed herein are linked by means of an interposed peptide linker comprising one or more amino acids. Such linkers may provide desirable flexibility to permit the desired expression, activity and/or conformational positioning of the chimeric polypeptide. A typical amino acid linker is generally designed to be flexible or to interpose a structure, such as an alpha-helix, between the two protein moieties. A linker can be fused to the N-terminus or C-terminus of a polypeptide encoding a lysosomal enzyme, or inserted internally. A linker is also referred to as a spacer.

The linker peptide sequence is of appropriate length to connect one or more proteins of interest and is preferably designed to be sufficiently flexible so as to allow the proper folding and/or function and/or activity of one or both of the peptides it connects. Thus, the linker peptide can have a length of no more no more than 20, no more than 25, no more than 30, no more than 35, no more than 40, no more than 45, no more than 50, no more than 55, no more than 60, no more than 65, no more than 70, no more than 75 or no more than 80amino acids. In some embodiments, the linker peptide can have a length of at least 18, at least 20, at least 25, at least 30, at least 35, at least 40, at least 45, or at least 50 amino acids. In some embodiments, the linker comprises at least 10 and no more than 60 amino acids, at least 10 and no more than 55 amino acids, at least 10 and no more than 50 amino acids, at least 10 and no more than 45 amino acids, at least 10 and no more than 40 amino acids, at least 10 and no more 35 amino acids, at least 10 and no more than 30 amino acids, at least 10 and no more than 25 amino acids, or at least 10 and no more than 20 amino acids. In certain embodiments, the linker comprises 12 to 57 amino acids, and in particular embodiments, comprises 57 amino acids. In a polypeptide composition comprising a linker, the N-terminus of the linker peptide sequence (e.g., amino acid sequence) is adjacent to and covalently linked to the C-terminus of one protein sequence (e.g., full-length protein or protein domain, fragment or variant) and, further, the C-terminus of the linker amino acid sequence is adjacent to and covalently linked to the N-terminus of another protein sequence. Polypeptide compositions produced in this manner are commonly referred to a fusion or chimeric protein/polypeptides and typically are made by the expression (e.g., transcription, translation) of nucleic acid sequences encoding the polypeptide compositions, in the appropriate system. Means by which to make fusion and/or chimeric polypeptides are well-known in the art (see for example, Sambrook et al., Molecular Cloning: A Laboratory Manual, Cold Springs Harbor Laboratory,1992) New York).

The inventors have discovered that simple amino acids (e.g., amino acids with simple side chains (e.g., H, CH3 or CH₂OH) and/or unbranched) are advantageous for use in a peptide linker as the lack of branched side chains on these amino acids provides greater flexibility (e.g., two-dimensional or three-dimensional flexibility) within the linker and, accordingly, within a polypeptide composition. Further, the inventors have found that alternating the glycine, alanine and/or serine residues provides even more order and greater flexibility with in the linker.

In addition, the inventors have discovered that placing a proline residue within the first (e.g., N-terminus) and/or last (e.g., C-terminus) five amino acids of the linker provides for additional benefit within the linker. Not to be bound by theory, it is believed that, unlike glycine, alanine and serine which are flexible amino acids, proline, whose cyclic side chain results in inflexibility, may result in a kink near the end(s) of the otherwise flexible linker, and thereby keep the polypeptides connected by the linker appropriately separated. Thus, the linker amino acid sequence has a proline in the third amino acid residue and optionally a proline within the last amino acid residue within the linker. In certain embodiments, the peptide linker can comprise 18 contiguous amino acid residues in which one of the amino acid residues is a proline that is located at the third amino residue of the linker, and the remaining 17 amino acid residues are comprised of glycine and alanine residues. The linker can further comprise three contiguous amino acids comprising a second proline, a glycine residue and an alanine residue, to produce a peptide linker comprising 21 amino acids. The second proline may also be the last amino acid in the linker amino acid sequence.

In some embodiments, the glycine alanine glycine amino acid repeats of the linker are flanked by glycine alanine proline (GAP) amino acid sequences at the C-terminus of the linker amino acid sequence (e.g., SEQ ID NO. 2). The GAP sequence in this instance is encoded for by nucleic acid sequence that represents an AscI restriction endonuclease site. In some embodiments, the linker amino acid sequence consists of SEQ ID NO. 1 (GAPGGGGGAAAAAGGGGG). In other embodiments, the linker amino acid sequence consists of one to four(e.g., 1, 2, 3, or 4) sequential repeats of SEQ ID NO. 1. The inventors have discovered that even one repeat of SEQ ID NO. 1 improves the linker functionality with three and four repeats of SEQ ID NO. 1 being most effective in allowing expression and/or activity of the linked polypeptides. In certain embodiments, the one to four sequential repeats of SEQ ID NO. 1 are further comprised of a GAP sequence at the C-terminus of the linker's amino acid sequence. In some embodiments, the linker amino acid sequence consists of SEQ ID NO. 2 (GAPGGGGGAAAAAGGGGGGGAPGGGGGAAAAAGGGGGGAPGGGGGAAAAAGGGGGGA P) .

In the polypeptide compositions described herein, the two polypeptides (e.g., a first polypeptide and a second polypeptide) can be recombinantly joined by any of the linker polypeptides described above, with the linker disposed between the two polypeptides. For example, in certain embodiments, the polypeptides or compositions comprise a first and a second polypeptide recombinantly joined by a linker consisting of SEQ ID NO. 1 or SEQ ID NO. 2. The two polypeptides can be any amino acid sequences including full-length proteins, protein fragments or portions, functional protein fragments or portions, functional protein domains and the like, of either two different proteins or the same protein. As used herein, "functional fragment" or "portion" is intended to refer to less than the entire mature or native protein which is sufficient to retain one or more of the desired biological activities of the mature or native protein (e.g., sufficient to retain a therapeutic or ameliorative biological activity with respect to a disorder to be treated). Thus, amino acid sequences or polypeptides can be modified, for example, polypeptide sequences into which amino acids have been inserted, deleted and/or substituted in such a manner that the modifications do not substantially interfere with the polypeptide's ability to encode a functional agent.

In some embodiments, one protein of the polypeptide composition is a peptide having a desired activity, while the other polypeptide delivers or targets the polypeptide having a desired activity to a specific cell or tissue. As used herein, the phrase targeting ligand or binding peptide refers to an amino acid sequence which serves to direct and deliver an agent (e.g., protein, polypeptide) to a specific site for the desired activity. In particular embodiments, the desired activity of one of the polypeptides is a therapeutic or prophylactic activity (e.g., treatment, replacement, inhibition, prevention, enhancement, reduction or amelioration). For example, in some embodiments, the polypeptide compositions described herein comprise one or more enzymes and/or proteins that are deficient in a lysosomal storage disease/disorder. For instance, the disclosed compositions may comprise one or more therapeutic agents comprising or consisting of an amino acid sequence derived from one or more of aspartylglucosaminidase, acid lipase, cysteine transporter, Lamp-2, α-galactosidase A, lipoprotein lipase LPL), ceramidase, α-L-fucosidase, β-hexosaminidase A, β-glucoronidase, GM2 ganglioside activator protein, α-D- mannosidase, β-D-mannosidase, arylsulphatase A, saposin B, neuraminidase, α-N-acetylglucosaminidase, phosphotransferase, phosphotransferase, L-iduronidase, iduronate-2-sulphatase, idursulfase, heparan-N-sulphatase, heparin sulfamidase, α-N-acetylglucosaminidase, N- acetyltransferase, N-acetylglucosamine 6-sulphatase, galactose 6-sulphatase, β-galactosidase, N- acetylgalactosamine 4-sulphatase, N-acetylglucosamine 6- sulfatase, hyalurono-glucosaminidase, multiple sulphatases, palmitoyl protein thioesterase, tripeptidyl peptidase I, acid sphingomyelinase, α-galactosidase B, sialic acid, and functional fragments, subunits and combinations of the above. In certain embodiments, one of the proteins (e.g., the therapeutic protein) of a polypeptide composition comprises N-acetyl-alpha- glucosaminidase (NaGlu), particularly human NaGlu or a functional portion, fragment, variant, mutant or derivative of NaGlu. Loss of the lysosomal enzyme NaGlu is believed to be responsible for the lysosomal storage disorder, Sanfilippo syndrome.

In some embodiments, one of the polypeptides of the polypeptide composition comprises a cell-surface receptor ligand and, in particular embodiments, the polypeptide is IGFII, one of the ligands of the IGFII/cation-independent mannose 6-phosphate receptor (IGFII/CI-MPR). The IGFII/CI-MPR recognizes mannose 6-phosphate (Man6-P) moieties added to oligosaccharides on newly synthesized lysosomal enzymes in mammalian cells. As the Man6-P interaction with the IGFII/CI-MPR regulates normal intracellular trafficking that brings newly synthesized enzymes to the lysosome, IGFII/CI-MPR is thought to be a receptor mechanism that could be used to deliver the lysosomal enzymes to cells. The above-described compositions would then rely on receptor-mediated transcytosis mechanisms to deliver the linked protein (e.g., therapeutic protein) to the cell of interest (e.g., endothelial cells, macrophage or neuronal cells). Physiologically, receptor-mediated transport is relied upon to transport macromolecules (e.g., proteins, polypeptides) into the cell, and generally involves ligand-recognition and binding of a macromolecule (e.g., and IGFI or IGFII moiety) to a specific receptor binding domain (e.g., the cation-independent mannose-6 phosphate receptor (CI-MPR), the IGFI receptor, the IGFII receptor or the IGFII/CI-MPR) on the targeted cells. Following recognition and binding of the ligand to the binding domain on the receptor, the receptor-ligand complex undergoes endocytosis by the cell (e.g., endothelial cells or macrophage) and the complex is thereby internalized. The ligand may then be transported across the abluminal membrane of the cell (e.g., an endothelial cell, a neuronal cell, a glial cell, a perivascular cell and/or a meningeal cell) and into the appropriate tissue (e.g., tissues of the central nervous system such as brain or spinal tissue). In certain embodiments described herein, a binding or targeting peptide of a polypeptide composition comprises SEQ ID NO. 4, amino acid residues 8 through 67 of IGFII.

Also contemplated herein is the inclusion of functional protein labels or tags into the disclosed compositions to provide additional means of isolating and/or detecting the translated polypeptide or protein. Suitable labels and tags are well known in the art and, for example, include, but are not limited to luciferase, green fluorescent protein, alkaline phosphatase, horseradish peroxidase, myc-tags, FLAG tags, eTags and polyhistidine tags. In a preferred embodiment, such labels and tags are capable of providing a detectable signal to facilitate identification of such labels or tags, for example upon distribution of the amino acid sequence encoding the polypeptide composition into the desired cells and tissues (e.g., CNS tissue).

The polypeptide compositions described herein which comprise two polypeptides connected by a linker sequence, can be synthetically produced (e.g., by chemical synthesis) or encoded for and expressed by (e.g., transcribed and translated) a polynucleotide (e.g., nucleic acid) sequence. As used herein, a "polynucleotide" refers to contiguous, covalently linked nucleic acid or nucleic acid molecules, such as deoxyribonucleic acid (DNA) or ribonucleic acid (RNA), oligonucleotides, fragments generated by any appropriate means known in the art (e.g., ligation or polymerase chain reaction (PCR)), and/or fragments generated by any of ligation, scission, endonuclease action, and exonuclease action. Polynucleotide molecules can be composed of monomers that are naturally-occurring nucleotides (such as DNA and RNA). In some embodiments, the polypeptide compositions described herein are encoded by the homologous polynucleotide sequences. The polynucleotides are produced using recombinant DNA technique, known to those with skill in the art (see, e.g., Sambrook et al., 1992). Generally, in accordance with the present invention, one or more targeting agents (e.g., a ligand/binding protein like IGFII or a biologically active fragment thereof) are operably linked to a nucleic acid or an amino acid sequence encoding a therapeutic agent (e.g., the enzyme NaGlu or a biologically active fragment thereof). In some of the polynucleotide molecules described herein, comprised of nucleotide sequences of at least two genes joined by a linker sequence, can produce fusion or chimeric polypeptides that may represent a hybrid of the two proteins. The polypeptide compositions described herein may also comprise suitable regulatory elements which can be cloned into an expression vector and expressed in a suitable host. Recombinant methods for designing, expressing and purifying fusion proteins are known in the art (see, e.g. Sambrook, et al., 1992).

Also contemplated herein are expression vectors containing the above-described polynucleotides and recombinant cells comprising the polynucleotides or expression vectors. An "expression vector" is a polynucleotide molecule encoding a gene that is expressed in a host cell. Typically, an expression vector comprises a transcription promoter, a gene, and a transcription terminator. Gene expression is usually placed under the control of a promoter, and such a gene is said to be "operably linked to" the promoter. Similarly, a regulatory element and a promoter can be operably linked if the regulatory element modulates the activity of the promoter. A "recombinant" or "host" cell used for expression of a vector is a cell that contains a polynucleotide molecule, such as the polynucleotides described herein. Large amounts of proteins may be produced *in vitro* using such expression vectors and/or recombinant cells and, accordingly, contemplated herein are methods of producing the disclosed polypeptide compositions. Such methods involve culturing a recombinant and/or host cell comprising a polynucleotide (e.g., expression vector) under conditions suitable for expression of the polypeptide from the polynucleotide. Any cell with protein synthetic capacity may be used for this purpose (e.g., animal, bacterial, yeast or insect cells). If a particular protein modification is required, animal cells and, in particular, mammalian cells may be necessary. Cells that may be used to express the polypeptide compositions include, but are not limited to, HT1080, HF1156, Chinese hamster ovary (CHO) cells, CHO-Kl cells, HeLa cells, Vero cells, FAO (liver cells), human 3T3 cells, A20 cells, EL4 cells, HepG2 cells, J744A cells, Jurkat cells, P388Dl cells, RC-4B/c cells, SK-N-SH cells, Sp2/mIL-6 cells, SW480 cells, 3T6 Swiss cells and the like. Suitable conditions for protein expression in various cells/systems are dependent on the cells/system and well-known in the art (Sambrook et al., 1992).

Also contemplated are pharmaceutical compositions that can be administered to a subject (e.g., a subject with a disease or disorder) to achieve a desired therapeutic effect (e.g., distribution into the cells and tissues of interest). Pharmaceutical compositions contemplated herein include, for example, nucleic acid or amino acid sequences encoding one or more therapeutic agents (e.g., the lysosomal enzyme NaGlu), operably linked to one or more targeting ligands (e.g., a fragment of IGFII), or vector or cells comprising the nucleic acid or amino acid sequences. Such amino or nucleic acids may be administered alone, but are preferably administered in combination with at least one other agent or excipient (e.g., a pharmaceutically-acceptable carrier such as buffered saline, dextrose, and purified water).

Suitable pharmaceutically-acceptable carriers preferably stabilize the proteins, enzymes, nucleic acids, amino acids and/or polypeptides suspended or solubilized therein and facilitate the processing of such proteins, enzymes, nucleic acids, amino acids and/or polypeptides into pharmaceutical compositions which may be administered to a subject. The described pharmaceutical compositions can be administered by any number of routes including, but not limited to, oral, intravenous, intramuscular, intra- arterial, intramedullary, intrathecal, intraventricular, transdermal, subcutaneous, intraperitoneal, intranasal, parenteral, topical, sublingual, or rectal means.

Pharmaceutical formulations suitable for parenteral administration can be formulated in aqueous solutions, preferably in physiologically compatible buffers such as physiologically buffered saline. Aqueous injection suspensions can contain substances which increase the viscosity of the suspension, such as sodium carboxymethyl cellulose, sorbitol, or dextran. Optionally, the suspension also can contain suitable stabilizers or agents which increase the solubility of the compounds to allow for the preparation of highly concentrated solutions. Further details on techniques for formulation and administration can be found in the latest edition of Remington's Pharmaceutical Science (Maack Publishing Co., Easton, Pa.). After pharmaceutical compositions have been prepared, they can be placed in an appropriate container and labeled for treatment of an indicated condition (e.g., for the treatment of Sanfilippo syndrome). Such labeling may include, but not be limited to instructions to calculate an effective amount of the pharmaceutical composition to be administered to the subject, appropriate dosing schedules, acceptable routes of administration and anticipated adverse effects.

Also contemplated is the delivering of a therapeutic polypeptide to a subject in need thereof by administering the polypeptide compositions and/or expression vectors and/or recombinant cells disclosed herein, to a subject in need thereof. Further contemplated is methods of treating a lysosomal storage disorder (e.g., Sanfilippo syndrome) by administering an effective amount of the disclosed polypeptide compositions to a subject in need thereof. As used herein, the term "subject" is meant to refer to any mammal (e.g., human, mouse, rat, dog, cat, pig, monkey, horse), particularly humans. In certain embodiments, the subject is an adult, an adolescent or an infant. Also contemplated is the administration of the compositions and/or performance of the methods of treatment in-utero. The compositions disclosed herein may be administered using any of the above-described routes of administration and, in certain embodiments, the polypeptide compositions are administered parenterally.

As used herein, the phrase "effective amount" refers to the amount of therapeutic agent and/or polypeptide needed to achieve a desired clinical effect and is largely determined based on the total amount of the therapeutic agent contained in a pharmaceutical composition. Generally, an effective amount of a therapeutic agent is sufficient to achieve a meaningful benefit to the subject (e.g., treating, modulating, curing, preventing and/or ameliorating the underlying disease or condition). For example, an effective amount of a pharmaceutical composition described herein may be an amount sufficient to achieve a desired therapeutic and/or prophylactic effect, such as an amount sufficient to modulate lysosomal enzyme receptors or their activity to thereby treat such lysosomal storage disorder or the symptoms thereof.

Generally, the amount of a therapeutic agent (e.g., a recombinant lysosomal enzyme) administered to a subject in need thereof will depend upon the characteristics of the subject. Such characteristics include the condition, general health, age, sex and body weight of the subject. One of ordinary skill in the art will be readily able to determine the appropriate dosages depending on these and other related factors. In addition, both objective and subjective assays may optionally be employed to identify optimal dosage ranges.

### EXEMPLIFICATION

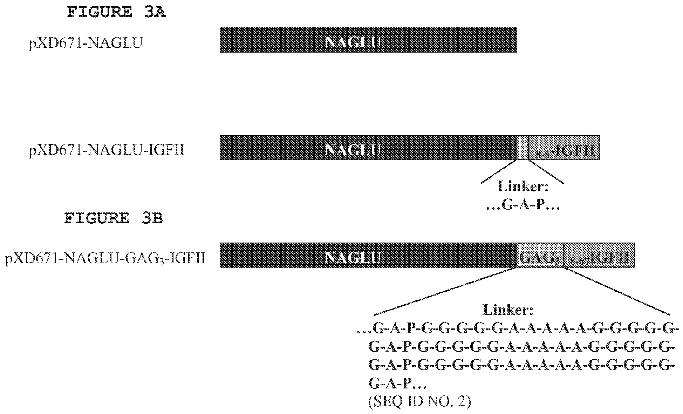

To maximize uptake of the lysosomal enzyme N-acetyl-alpha-glucosaminidase (Naglu), a cassette encoding residues 8-67 of the mature IGFII was fused in frame to the C-terminus of the full length human Naglu open reading frame. The design of this construct was similar glucoronidase-IGFII fusion protein described by LeBowitz et al. (PNAS 101(9):3083-3088, 2004), who observed that cellular uptake of glucoronidase, through the IGFII cation-independent mannose-6-phosphate receptor, was greatly improved when fused to IGFII, which binds with high affinity to a distinct site on the receptor. To establish the benefit of such a fusion protein, two expression plasmids were generated (FIG. 3A), one expressing full length wild-type human Naglu and the other expressing the Naglu-IGFII fusion protein with a linker region consisting of 3 residues, glycine alanine proline (GAP) (LeBowitz, 2004).

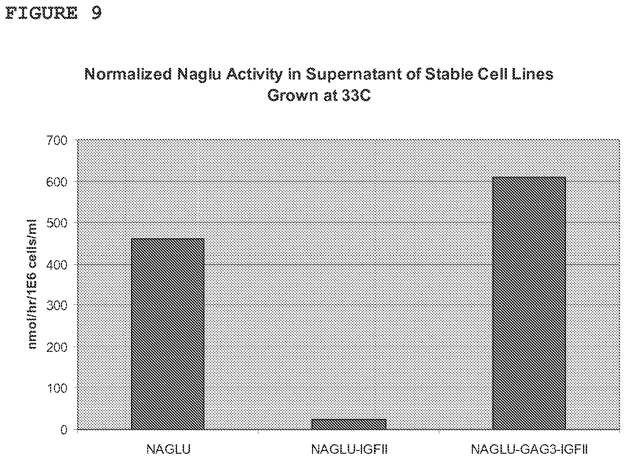

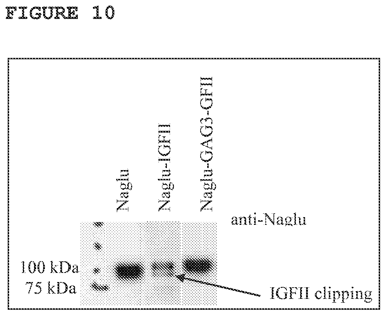

HT1080 mammalian stable cell lines were generated for both wild-type Naglu and Naglu-IGFII. Following seeding of stable cell lines at 1x10⁶ cells/ml and culture at 33°C for 24 hours, protein expression was monitored in the conditioned media by western blot and activity determined by measuring the cleavage of the fluorogenic substrate 4MU-N-acetyl-alpha-D-glucosaminide. It was determined that activity, normalized by cell number, of Naglu-IGFII was 10 fold lower than that observed for untagged Naglu (see FIG. 9). By western blot, when sample load was normalized by cell number, it was apparent that Naglu- IGFII expression was significantly less than untagged Naglu (FIG. 10). Furthermore, there was also a significant amount of degradation occurring during Naglu-IGFII expression as evidenced by the lower molecular weight band running beneath the upper band in the Naglu- IGFII lane of the western blot (arrow, FIG. 10). This level of expression and breakdown was observed in all Naglu-IGFII stable clones developed which encompassed 3 separate stable transfections. In fact, when analyzing expression level and activity of various Naglu-IGFII clones, there was a very obvious correlation between the amount of degradation observed by western blot and the level of Naglu-IGFII activity in the sample. Thus, clones displaying higher levels of Naglu-IGFII in the conditioned media typically had a corresponding higher level of degradation on western blots. It was suspected that the Naglu-IGFII was relatively inactive due to the placement of the IGFII protein, and that the level of activity observed in samples taken from Naglu-IGFII clones was primarily due to clipping of the IGFII protein, resulting in an increased level of active, untagged Naglu in the cell-conditioned media samples.

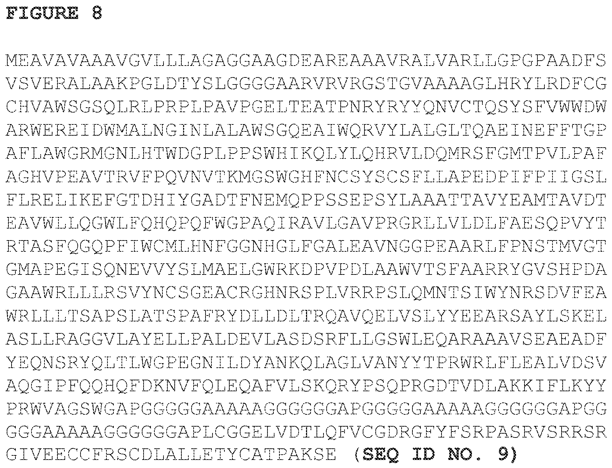

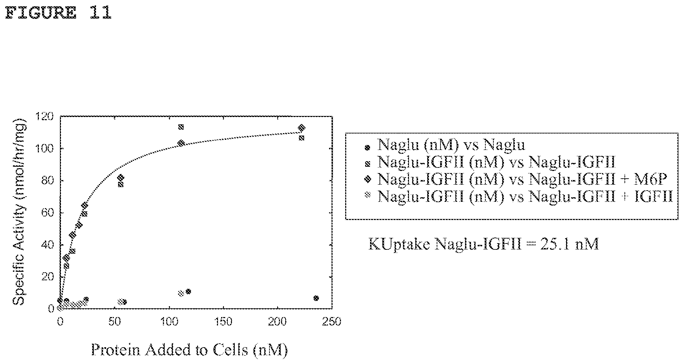

It was hypothesized that increasing the linker length between Naglu and IGFII might improve the expression and activity of the Naglu-IGFII fusion protein by allowing for a more conformationally stable folded protein and/or prevent any potential interference between IGFII and the Naglu active site. To increase the linker length, complementary oligos were generated consisting of glycine alanine glycine repeats flanked by the original gap linker, which is encoded by an AscI restriction endonuclease site. The oligo was then ligated into the AscI site. In this ligation, 3 gag oligos were incorporated into the linker, resulting in a linker that was 57 amino acids long (FIG. 3B and FIG. 8). Stable clones generated from this construct yielded Naglu-GAG₃- IGFII protein that was as active (FIG. 9) and expressed to similar levels (FIG. 10) as Naglu. This confirmed that a longer linker allowed for proper folding and enzymatic activity of the Naglu-IGFII fusion protein. Furthermore, recombinant Naglu-GAG₃-IGFII and Naglu were tested for uptake in human fibroblast cells (HF1156). Naglu-GAG₃-IGFII was readily taken up by the cells, and that uptake was inhibited by IGFII but not mannose 6-phosphate (M6P), in a dose-dependent manner (FIG. 11). As expected, recombinant Naglu, lacking M6P, showed no uptake in the cells (FIG. 11).

Thus, the inventors surprisingly discovered a peptide linker which resulted in a highly expressed and active NaGlu-IGFII polypeptide. From this work, it is believed that a longer linker (e.g., longer than 3 amino acids), resulted in the proper folding/activity of the protein and prevented its degradation. Accordingly, this and similar peptide linkers can be used generally to link other proteins and create heterologous polypeptides.

While certain compositions and methods of the present invention have been described with specificity in accordance with certain embodiments, the above examples serve only to illustrate the compounds of the invention and are not intended to limit the same.

## Claims

1. A polypeptide composition comprising:
a) a first peptide;
b) a second peptide; and
c) a linker consisting of one to four sequential repeats of the amino acid sequence of SEQ ID NO. 1, and optionally the amino acid sequence glycine alanine proline (GAP) at the C-terminus, wherein the linker is disposed between said first peptide and said second peptide.

2. The polypeptide composition of claim 1, wherein the first peptide is a therapeutic agent and the second peptide is a targeting agent, optionally wherein:
a) the therapeutic agent is a lysosomal protein or an enzyme; and
b) the targeting agent is a cell surface receptor ligand protein.

3. The polypeptide composition of claim 1 or claim 2, wherein (a) said linker consists of one to four sequential repeats of SEQ ID NO. 1 and the amino acid sequence glycine alanine proline (GAP) at the C-terminus, optionally wherein the linker consists of the amino acid sequence of SEQ ID NO. 2; and/or (b) said first peptide comprises the amino acid sequence of SEQ ID NO. 4; and/or (c) said second peptide comprises a receptor binding domain; and/or (d) said second peptide comprises the amino acid sequence of SEQ ID NO. 6.

4. The polypeptide composition of any one of claims 1 to 3, wherein:
a) the first peptide comprises the amino acid sequence of SEQ ID NO. 4; and
b) the second peptide comprises the amino acid sequence of SEQ ID NO. 6; optionally wherein said linker consists of one to four sequential repeats of SEQ ID NO. 1 and the amino acid sequence glycine alanine proline (GAP) at the C-terminus.

5. The polypeptide composition of claim 4, wherein the linker consists of the amino acid sequence of SEQ ID NO. 2.

6. A polypeptide linker composition consisting of one to four sequential repeats of the amino acid sequence of SEQ ID NO: 1 (GAPGGGGGAAAAAGGGGG) and optionally the amino acid sequence glycine alanine proline (GAP) at the C-terminus.

7. The polypeptide linker composition of claim 6 consisting of one to four sequential repeats of the amino acid sequence of SEQ ID NO:1 and the amino acid sequence glycine alanine proline (GAP) at the C-terminus, optionally wherein the polypeptide linker composition is of the amino acid sequence of SEQ ID NO. 2.

8. A polynucleotide encoding a polypeptide linker consisting of the amino acid sequence of one to four sequential repeats of SEQ ID NO. 1 and optionally the amino acid sequence glycine alanine proline (GAP) at the C-terminus, optionally wherein the linker consists of the amino acid sequence of SEQ ID NO. 2.

9. The polynucleotide according to claim 8 encoding a polypeptide composition comprising the amino acid sequence of:
a) a first peptide;
b) a second peptide; and
c) the linker being disposed between said first peptide and said second peptide; optionally wherein (a) said linker of said polypeptide composition consists of one to four sequential repeats of SEQ ID NO. 1 and the amino acid sequence glycine alanine proline (GAP) at the C-terminus; optionally wherein said linker of said polypeptide composition consists of SEQ ID NO. 2 (b) said first peptide of said polypeptide composition comprises the amino acid sequence of SEQ ID NO. 4; and/or (c) said second peptide of said polypeptide composition comprises a receptor binding domain.

10. The polynucleotide of claim 9, wherein said second peptide comprises the amino acid sequence of SEQ ID NO. 6.

11. An expression vector comprising a polynucleotide of claim 9 or claim 10.

12. A recombinant cell comprising a polynucleotide of claim 9 or claim 10, or the expression vector of claim 11.

13. A pharmaceutical composition comprising a polypeptide composition of claims 1 to 5 and a pharmaceutically acceptable carrier; or a polynucleotide of claim 9 or claim 10 and a pharmaceutically acceptable carrier; or a recombinant cell of claim 12 and a pharmaceutically acceptable carrier.

14. A method of producing a polypeptide composition comprising culturing a recombinant cell of claim 12 under conditions suitable for expression of said polypeptide.

15. The polypeptide composition of any one of claims 1 to 5, wherein the first peptide is a therapeutic agent and the second peptide is a targeting agent for use in delivering a therapeutic polypeptide to a subject in need thereof.

16. A polypeptide composition according to any one of claims 1 to 5, comprising:
a) a first peptide comprising the amino acid sequence of SEQ ID NO. 4;
b) a second peptide comprising the amino acid sequence of SEQ ID NO. 6; and
c) a linker consisting of one to four sequential repeats of the amino acid sequence of SEQ ID NO. 1 and optionally the amino acid sequence glycine alanine proline (GAP) at the C-terminus, wherein the linker is disposed between said first peptide and said second peptide
for use in delivering a therapeutic polypeptide to a subject in need thereof.

17. The polypeptide composition for use according to claim 16, wherein the linker consists of one to four sequential repeats of the amino acid sequence of SEQ ID NO:1 and the amino acid sequence glycine alanine proline (GAP) at the C-terminus, optionally wherein the linker consists of the amino acid sequence of SEQ ID NO. 2.

18. One or more expression vectors of claim 11, or one or more recombinant cells of claim 12 for use in delivering a therapeutic polypeptide to a subject in need thereof, wherein the first peptide is a therapeutic agent and the second peptide is a targeting agent.

19. An effective amount of the pharmaceutical composition of claim 13, wherein the first peptide is a lysosomal enzyme and the second peptide is a cell surface receptor ligand protein, for use in treating a lysosomal storage disease; optionally wherein said lysosomal storage disease is Sanfilippo syndrome.

20. A pharmaceutical composition for use in treating Sanfilippo syndrome comprising a polypeptide composition comprising:
a) a first peptide comprising the amino acid sequence of SEQ ID NO. 4;
b) a second peptide comprising the amino acid sequence of SEQ ID NO. 6; and
c) a linker consisting of one to four sequential repeats of the amino acid sequence of SEQ ID NO. 1, and optionally the amino acid sequence glycine alanine proline (GAP) at the C-terminus, wherein the linker is disposed between said first peptide and said second peptide.

21. The pharmaceutical composition for use according to claim 20, wherein the linker consists of one to four sequential repeats of the amino acid sequence of SEQ ID NO. 1 and the amino acid sequence glycine alanine proline (GAP) at the C-terminus, optionally wherein the linker consists of the amino acid sequence of SEQ ID NO. 2.

22. The pharmaceutical composition for use according to any one of claims 19 to 21 wherein (a) said pharmaceutical composition is administered parenterally; or (b) said pharmaceutical composition is administered orally.

## Patentansprüche

1. Polypeptidzusammensetzung, Folgendes umfassend:
a) ein erstes Peptid;
b) ein zweites Peptid; und
c) einen Linker, bestehend aus einem bis vier sequentiellen Repeats der Aminosäuresequenz von SEQ ID NO. 1 und optional der Aminosäuresequenz Glycin-Alanin-Prolin (GAP) am C-Terminus, wobei der Linker zwischen dem ersten Peptid und dem zweiten Peptid angeordnet ist.

2. Polypeptidzusammensetzung nach Anspruch 1 wobei das erste Peptid ein Therapeutikum ist und das zweite Peptid ein Targeting-Mittel ist, optional wobei:
a) das Therapeutikum ein lysosomales Protein oder ein Enzym ist; und
b) das Targeting-Mittel ein Zelloberflächenrezeptorligandenprotein ist.

3. Polypeptidzusammensetzung nach Anspruch 1 oder 2, wobei (a) der Linker aus einem bis vier sequentiellen Repeats von SEQ ID NO. 1 und der Aminosäuresequenz Glycin-Alanin-Prolin (GAP) am C-Terminus besteht, optional wobei der Linker aus der Aminosäuresequenz von SEQ ID NO. 2 besteht; und/oder (b) das erste Peptid die Aminosäuresequenz von SEQ ID NO: 4 umfasst; und/oder (c) das zweite Peptid eine Rezeptorbindungsdomäne umfasst; und/oder (d) das zweite Peptid die Aminosäuresequenz von SEQ ID NO: 6 umfasst.

4. Polypeptidzusammensetzung nach einem der Ansprüche 1 bis 3, wobei:
a) das erste Peptid die Aminosäuresequenz von SEQ ID NO: 4 umfasst; und
b) das zweite Peptid die Aminosäuresequenz von SEQ ID NO: 6 umfasst;
optional wobei der Linker aus einem bis vier sequentiellen Repeats von SEQ ID NO. 1 und der Aminosäuresequenz Glycin-Alanin-Prolin (GAP) am C-Terminus besteht.

5. Polypeptidzusammensetzung nach Anspruch 4, wobei der Linker aus der Aminosäuresequenz von SEQ ID NO: 2 besteht.

6. Polypeptidlinkerzusammensetzung, bestehend aus einem bis vier sequentiellen Repeats der Aminosäuresequenz von SEQ ID NO. 1 (GAPGGGGGAAAAAGGGGG) und optional der Aminosäuresequenz Glycin-Alanin-Prolin (GAP) am C-Terminus.

7. Polypeptidlinkerzusammensetzung nach Anspruch 6, bestehend aus einem bis vier sequentiellen Repeats der Aminosäuresequenz von SEQ ID NO. 1 und der Aminosäuresequenz Glycin-Alanin-Prolin (GAP) am C-Terminus, optional wobei die Polypeptidzusammensetzung aus der Aminosäuresequenz von SEQ ID NO. 2 besteht.

8. Polynukleotid, das für einen Polypeptidlinker kodiert, bestehend aus der Aminosäuresequenz von einem bis vier sequentiellen Repeats von SEQ ID NO. 1 und optional der Aminosäuresequenz Glycin-Alanin-Prolin (GAP) am C-Terminus, optional wobei der Linker aus der Aminosäuresequenz von SEQ ID NO. 2 besteht.

9. Polynukleotid nach Anspruch 8, das für eine Polypeptidzusammensetzung kodiert, umfassend die Aminosäuresequenz von
a) einem ersten Peptid;
b) einem zweiten Peptid; und
c) wobei der Linker zwischen dem ersten Peptid und dem zweiten Peptid angeordnet ist;
optional wobei (a) der Linker der Polypeptidzusammensetzung aus einem bis vier sequentiellen Repeats von SEQ ID NO. 1 und der Aminosäuresequenz Glycin-Alanin-Prolin (GAP) am C-Terminus besteht; optional wobei der Linker der Polypeptidzusammensetzung aus SEQ ID NO. 2 besteht; (b) das erste Peptid der Polypeptidzusammensetzung die Aminosäuresequenz von SEQ ID NO: 4 umfasst; und/oder (c) das zweite Peptid der Polypeptidzusammensetzung eine Rezeptorbindungsdomäne umfasst.

10. Polynukleotid nach Anspruch 9, wobei das zweite Peptid die Aminosäuresequenz von SEQ ID NO: 6 umfasst.

11. Expressionsvektor, ein Polynukleotid nach Anspruch 9 oder 10 umfassend.

12. Rekombinante Zelle, umfassend ein Polynukleotid nach Anspruch 9 oder 10 oder den Expressionsvektor nach Anspruch 11.

13. Pharmazeutische Zusammensetzung, umfassend eine Polypeptidzusammensetzung nach Anspruch 1 bis 5 und einen pharmazeutisch annehmbaren Träger; oder ein Polynukleotid nach Anspruch 9 oder 10 und einen pharmazeutisch annehmbaren Träger; oder eine rekombinante Zelle nach Anspruch 12 und einen pharmazeutisch annehmbaren Träger.

14. Verfahren zum Erzeugen einer Polypeptidzusammensetzung, umfassend das Kultivieren einer rekombinanten Zelle nach Anspruch 12 unter Bedingungen, die geeignet sind zum Exprimieren des Polypeptids.

15. Polypeptidzusammensetzung nach einem der Ansprüche 1 bis 5, wobei das erste Peptid ein Therapeutikum ist und das zweite Peptid ein Targeting-Mittel zur Verwendung beim Verabreichen eines therapeutischen Polypeptids an ein Subjekt, das dies benötigt, ist.

16. Polypeptidzusammensetzung nach einem der Ansprüche 1 bis 5, Folgendes umfassend:
a) ein erstes Peptid, umfassend die Aminosäuresequenz von SEQ ID NO: 4;
b) ein zweites Peptid, umfassend die Aminosäuresequenz von SEQ ID NO: 6; und
c) einen Linker, bestehend aus einem bis vier sequentiellen Repeats der Aminosäuresequenz von SEQ ID NO. 1 und optional der Aminosäuresequenz Glycin-Alanin-Prolin (GAP) am C-Terminus, wobei der Linker zwischen dem ersten Peptid und dem zweiten Peptid angeordnet ist zur Verwendung beim Verabreichen eines therapeutischen Polypeptids an ein Subjekt, das dies benötigt.

17. Polypeptidzusammensetzung zur Verwendung nach Anspruch 16, wobei der Linker aus einem bis vier sequentiellen Repeats der Aminosäuresequenz von SEQ ID NO. 1 und der Aminosäuresequenz Glycin-Alanin-Prolin (GAP) am C-Terminus besteht, optional wobei der Linker aus der Aminosäuresequenz von SEQ ID NO. 2 besteht.

18. Ein oder mehrere Expressionsvektoren nach Anspruch 11, oder eine oder mehrere rekombinanten Zellen nach Anspruch 12 zur Verwendung beim Bereitstellen eines therapeutischen Polypeptids an ein Subjekt, das dies benötigt, wobei das erste Peptid ein Therapeutikum ist und das zweite Peptid ein Targeting-Mittel ist.

19. Wirksame Menge der pharmazeutischen Zusammensetzung nach Anspruch 13, wobei das erste Peptid ein lysosomales Enzym ist und das zweite Peptid ein Zelloberflächenrezeptorligandenprotein ist, zur Verwendung beim Behandeln einer lysosomalen Speicherkrankheit; optional wobei die lysosomale Speicherkrankheit das Sanfilippo-Syndrom ist.

20. Pharmazeutische Zusammensetzung zur Verwendung beim Behandeln von Sanfilippo-Syndrom, umfassend ein Polypeptid, das Folgendes umfasst:
a) ein erstes Peptid, umfassend die Aminosäuresequenz von SEQ ID NO: 4;
b) ein zweites Peptid, umfassend die Aminosäuresequenz von SEQ ID NO: 6; und
c) einen Linker, bestehend aus einem bis vier sequentiellen Repeats der Aminosäuresequenz von SEQ ID NO. 1 und optional der Aminosäuresequenz Glycin-Alanin-Prolin (GAP) am C-Terminus, wobei der Linker zwischen dem ersten Peptid und dem zweiten Peptid angeordnet ist.

21. Pharmazeutische Zusammensetzung zur Verwendung nach Anspruch 20, wobei der Linker aus einem bis vier sequentiellen Repeats der Aminosäuresequenz von SEQ ID NO. 1 und der Aminosäuresequenz Glycin-Alanin-Prolin (GAP) am C-Terminus besteht, optional wobei der Linker aus der Aminosäuresequenz von SEQ ID NO. 2 besteht.

22. Pharmazeutische Zusammensetzung zur Verwendung nach einem der Ansprüche 19 bis 21, wobei (a) die pharmazeutische Zusammensetzung parenteral verabreicht wird; oder (b) die pharmazeutische Zusammensetzung oral verabreicht wird.

## Revendications

1. Composition polypeptidique comprenant :
a) un premier peptide ;
b) un second peptide ; et
c) un lieur consistant en une à quatre répétitions séquentielles de la séquence d'acides aminés de SEQ ID NO: 1, et éventuellement en la séquence d'acides aminés glycine alanine proline (GAP) à l'extrémité C-terminale, dans laquelle le lieur est disposé entre ledit premier peptide et ledit second peptide.

2. Composition polypeptidique selon la revendication 1, dans laquelle le premier peptide est un agent thérapeutique et le second peptide est un agent de ciblage, éventuellement dans laquelle :
a) l'agent thérapeutique est une protéine lysosomale ou une enzyme ; et
b) l'agent de ciblage est une protéine ligand du récepteur de surface cellulaire.

3. Composition polypeptidique selon la revendication 1 ou la revendication 2, dans laquelle (a) ledit lieur consiste en une à quatre répétitions séquentielles de SEQ ID NO: 1 et en la séquence d'acides aminés glycine alanine proline (GAP) à l'extrémité C-terminale, éventuellement dans laquelle le lieur consiste en la séquence d'acides aminés de SEQ ID NO: 2 ; et/ou (b) ledit premier peptide comprend la séquence d'acides aminés de SEQ ID NO: 4 ; et/ou (c) ledit second peptide comprend un domaine de liaison au récepteur ; et/ou (d) ledit second peptide comprend la séquence d'acides aminés de SEQ ID NO: 6.

4. Composition polypeptidique selon l'une quelconque des revendications 1 à 3, dans laquelle :
a) le premier peptide comprend la séquence d'acides aminés de SEQ ID NO: 4 ; et
b) le second peptide comprend la séquence d'acides aminés de SEQ ID NO: 6 ;
éventuellement dans laquelle ledit lieur consiste en une à quatre répétitions séquentielles de SEQ ID NO: 1, et en la séquence d'acides aminés glycine alanine proline (GAP) à l'extrémité C-terminale.

5. Composition polypeptidique selon la revendication 4, dans laquelle le lieur consiste en la séquence d'acides aminés de SEQ ID NO: 2.

6. Composition de lieur polypeptidique, consistant en une à quatre répétitions séquentielles de la séquence d'acides aminés de SEQ ID NO: 1 (GAPGGGGGAAAAAGGGGG) et éventuellement en la séquence d'acides aminés glycine alanine proline (GAP) à l'extrémité C-terminale.

7. Composition de lieur polypeptidique selon la revendication 6, consistant en une à quatre répétitions séquentielles de la séquence d'acides aminés de SEQ ID NO: 1 et en la séquence d'acides aminés glycine alanine proline (GAP) à l'extrémité C-terminale, éventuellement dans laquelle la composition de lieur polypeptidique a la séquence d'acides aminés de SEQ ID NO: 2.

8. Polynucléotide codant pour un lieur polypeptidique consistant en la séquence d'acides aminés d'une à quatre répétitions de SEQ ID NO: 1 et éventuellement en la séquence d'acides aminés glycine alanine proline (GAP) à l'extrémité C-terminale, éventuellement dans laquelle le lieur consiste en la séquence d'acides aminés de SEQ ID NO: 2.

9. Polynucléotide selon la revendication 8, codant pour une composition polypeptidique comprenant la séquence d'acides aminés :
a) d'un premier peptide ;
b) d'un second peptide ; et
c) le lieur étant disposé entre ledit premier peptide et ledit second peptide
éventuellement dans lequel (a) ledit lieur de ladite composition polypeptidique consiste en une à quatre répétitions séquentielles de SEQ ID NO: 1 et en la séquence d'acides aminés glycine alanine proline (GAP) à l'extrémité C-terminale ; éventuellement dans lequel ledit lieur de ladite composition polypeptidique consiste en la SEQ ID NO: 2, (b) ledit premier peptide de ladite composition polypeptidique comprenant la séquence d'acides aminés de SEQ ID NO: 4 ; et/ou (c) ledit second peptide de ladite composition polypeptidique comprenant un domaine de liaison au récepteur.

10. Polynucléotide selon la revendication 9, dans lequel ledit second peptide comprend la séquence d'acides aminés de SEQ ID NO: 6.

11. Vecteur d'expression comprenant un polynucléotide selon la revendication 9 ou la revendication 10.

12. Cellule recombinante comprenant un polynucléotide selon la revendication 9 ou la revendication 10, ou le vecteur d'expression selon la revendication 11.

13. Composition pharmaceutique comprenant une composition polypeptidique selon les revendications 1 à 5 et un véhicule pharmaceutiquement acceptable ; ou un polynucléotide selon la revendication 9 ou la revendication 10 et un véhicule pharmaceutiquement acceptable ; ou une cellule recombinante selon la revendication 12 et un véhicule pharmaceutiquement acceptable.

14. Procédé de production d'une composition polypeptidique, comprenant la culture d'une cellule recombinante selon la revendication 12 dans des conditions convenant à l'expression dudit polypeptide.

15. Composition polypeptidique selon l'une quelconque des revendications 1 à 5, dans laquelle le premier peptide est un agent thérapeutique et le second peptide est un agent de ciblage pour une utilisation dans l'administration d'un polypeptide thérapeutique à un sujet en ayant besoin.

16. Composition polypeptidique selon l'une quelconque des revendication 1 à 5, comprenant :
a) un premier peptide comprenant la séquence d'acides aminés de SEQ ID NO: 4 ;
b) un second peptide comprenant la séquence d'acides aminés de SEQ ID NO: 6 ; et
c) un lieur consistant en une à quatre répétitions séquentielles de la séquence d'acides aminés de SEQ ID NO: 1 et éventuellement en la séquence d'acides aminés glycine alanine proline (GAP) à l'extrémité C-terminale, dans laquelle le lieur est disposé entre ledit premier peptide et ledit second peptide,
pour une utilisation dans l'administration d'un polypeptide thérapeutique à un sujet en ayant besoin.

17. Composition polypeptidique pour une utilisation selon la revendication 16, dans laquelle le lieur consiste en une à quatre répétitions séquentielles de la séquence d'acides aminés de SEQ ID NO: 1 et en la séquence d'acides aminés glycine alanine proline (GAP) à l'extrémité C-terminale, éventuellement dans laquelle le lieur consiste en la séquence d'acides aminés de SEQ ID NO: 2.

18. Un ou plusieurs vecteurs d'expression selon la revendication 11, ou une ou plusieurs cellules recombinantes selon la revendication 12, pour une utilisation dans l'administration d'un polypeptide thérapeutique à un sujet en ayant besoin, dans lesquels le premier peptide est un agent thérapeutique et le second peptide est un agent de ciblage.

19. Quantité efficace d'une composition pharmaceutique selon la revendication 13, dans laquelle le premier peptide est une enzyme lysosomale et le second peptide est une protéine ligand du récepteur de surface cellulaire, pour une utilisation dans le traitement de la maladie de stockage lysosomale ; éventuellement dans laquelle ladite maladie de stockage lysosomale est le syndrome de Sanfilippo.

20. Composition pharmaceutique pour une utilisation dans le traitement du syndrome de Sanfilippo, comprenant une composition polypeptidique comprenant :
a) un premier peptide comprenant la séquence d'acides aminés de SEQ ID NO: 4 ;
b) un second peptide comprenant la séquence d'acides aminés de SEQ ID NO: 6 ; et
c) un lieur consistant en une à quatre répétitions séquentielles de la séquence d'acides aminés de SEQ ID NO: 1, et éventuellement en la séquence d'acides aminés glycine alanine proline (GAP) à l'extrémité C-terminale, dans laquelle le lieur est disposé entre ledit premier peptide et ledit second peptide.

21. Composition pharmaceutique pour une utilisation selon la revendication 20, dans laquelle le lieur consiste en une à quatre répétitions séquentielles de la séquence d'acides aminés de SEQ ID NO: 1 et en la séquence d'acides aminés glycine alanine proline (GAP) à l'extrémité C-terminale, éventuellement dans laquelle le lieur consiste en la séquence d'acides aminés SEQ ID NO: 2.

22. Composition pharmaceutique pour une utilisation selon l'une quelconque des revendications 19 à 21, dans laquelle (a) ladite composition pharmaceutique est administrée par voie parentérale ; ou (b) ladite composition pharmaceutique est administrée par voie orale.
